(19) Europäisches Patentamt
European Patent Office
Office européen des brevets

(11) Numéro de publication: **0 018 884**
**B1**

# (12) FASCICULE DE BREVET EUROPEEN

(45) Date de publication du fascicule de brevet: **04.11.81**

(51) Int. Cl.³: **C 07 C 93/04**

(21) Numéro de dépôt: **80400523.9**

(22) Date de dépôt: **18.04.80**

## (54) Procéde de préparation de tris(éther-amines) et tris(éther-amines) ainsi obtenues.

(30) Priorité: **03.05.79 FR 7911100**

(43) Date de publication de la demande:
**12.11.80 Bulletin 80/23**

(45) Mention de la délivrance du brevet:
**04.11.81 Bulletin 81/44**

(84) Etats Contractants Désignés:
**AT BE CH DE GB IT LI LU NL SE**

(56) Documents cités:
**FR - A - 2 334 656**
**US - A - 2 285 419**

(73) Titulaire: **RHONE-POULENC INDUSTRIES**
**22, avenue Montaigne**
**F-75008 Paris (FR)**

(72) Inventeur: **Soula, Gérard**
**33, rue Nungesser**
**F-69330 - Meyzieu (FR)**
Inventeur: **Linguenheld, Louis**
**15, Chemin de Puttet**
**F-69230 - Saint-Genis Laval (FR)**

(74) Mandataire: **Fabre, Madeleine-France et al,**
**RHONE POULENC Service Brevets Chimie et**
**Polymères B.P.753**
**F-75360 Paris Cedex 08 (FR)**

**EP 0 018 884 B1**

Il est rappelé que: Dans un délai de neuf mois à compter de la date de publication de la mention de la délivrance du brevet européen toute personne peut faire opposition au brevet européen délivré. auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

# Procédé de préparation de tris(éther-amines) et tris(éther-amines) ainsi obtenues

La présente invention a pour objet un procédé de préparation de tris(éther-amines) de formule I

$$(I) \qquad N \left[ A - O \left( B - O \right)_n R \right]_3$$

où R représente:
- un radical alkyle contenant de 1 à 24 atomes de carbone
- un radical cyclohexyle
- un radical phényle
- un radical alkylphényle dont le groupe alkyle contient de 1 à 12 atomes de carbone;

A et B sont semblables ou différents et représentent un groupement alcanediyle linéaire contenant 2 ou 3 atomes de carbone, lesdits atomes de carbone pouvant être substitués par un radical méthyle ou éthyle;
n représente un nombre entier compris entre 0 et 4, ainsi que les tris(éther-amines) ainsi obtenues.

Il est connu d'après le brevet américain n° 2.285.419 de préparer des alcoxyamines de formule (A)

$$(A) \qquad {}_{3-m}H - N \left[ \left( CH_2 - CH_2O \right)_{1ou2} R \right]_m$$

où R représente un radical alcoyle inférieur;
m représente un nombre entier égal à 1, 2 ou 3,
par réaction de l'ammoniac sur le monoéther d'éthylène glycol correspondant en présence d'un catalyseur d'hydrogénation-déshydrogénation. Selon une variante du procédé, l'ammoniac peut être remplacé par une amine de formule A dans laquelle m est égal à 1 ou 2.

Un tel procédé nécessite de longues durées de réaction et ne permet d'aboutir qu'à de faibles rendements en alcoxyamines. Il ne peut donc être utilisé industriellement; en particulier, il n'est pas dou tout adapté à la préparation industrielle d'amines tertiaires de formule A dans laquelle m est égal à 3.

Il a été propsé (brevet français n° 1.302.365) d'améliorer les rendements en alcoxyamines en réalisant l'opération d'ammonolyse en phase vapeur et en opérant en outre en présence d'hydrogène. Si un tel procédé permet d'améliorer les rendements en alcoxyamines, il n'apporte par contre aucune solution quant à l'obtention d'amies tertiaires d'un manière selective.

Il a été proposé dans le brevet belge n° 849.348 de préparer d'une manière sélective des éther-amines secondaires de formule (B).

$$(B) \qquad HN \left[ \left( \overset{X}{\overset{|}{CH}} - \overset{Y}{\overset{|}{CH}} - O \right)_n R \right]_2$$

où R représente un radical alcoyle en $C_9$—$C_{24}$, cyclohexyle ou aryle, X et Y représentent un atome d'hydrogène ou un radical méthyle,
n représente un nombre entier compris entre 1 et 15,
par réaction en phase liquide du monoéther d'alkylèneglycol correspondant sur de l'ammoniac et de l'hydrogène, en présence d'un catalyseur d'hydrogénation-déshydrogénation à une température comprise entre 150 et 250°C sous une pression de 0,5 à 1,5 atomsphère et évacuation de l'eau réactionnelle avec le courant gazeux.

Ce brevet belge n° 849.348 décrit un procédé permettant d'obtenir des éther-amines secondaires avec une bonne sélectivité, mais n'indique nullement comment opérer pour orienter la réaction d'ammonolyse dans le sens de la formation d'amines tertiaires.

La demanderesse a trouvé un procédé pour préparer de manière sélective les tris(éther-amines) de formule (I) par ammonolyse des monoéthers d'alkylèneglycol correspondants.

Le procédé de préparation de tris(éther-amines) de formule I

$$(I) \qquad N \left[ A - O \left( B - O \right)_n R \right]_3$$

où R représente:

— un radical alkyle contenant de I à 24 atomes de carbone, de préférence de 1 à 12
— un radical cyclohexyle
— un radical phényle
— un radical alkylphényle dont le groupe alkyle contient de 1 à 12 atomes de carbone

A et B sont semblables ou différents et représentent un groupement alcanediyle linéaire contenant 2 ou 3 atomes de carbone, lesdits atomes de carbone pouvant être substitués par un radical méthyle ou éthyle n représente un nombre entier compris entre 0 et 4, de préférence entre 0 et 3.

par ammonolyse en phase liquide d'un monoéther d'alkylèneglycol de formule (II)

$$\text{(II)} \quad HO-A-O-(-B-O-)_n\,R$$

où R, A, B et n ont la définition donnée ci-dessus, à l'aide d'au moins un agent d'ammonolyse choisi parmi l'ammoniac et les éther-amines de formule (I')

$$\text{(I')} \quad {}_{3-p}(HN-)\left[A'-O-(-B'-O-)_{n'}\,R'\right]_p$$

où R', A', B' et n' sont respectivement indentiques à R, A, B et n et p est égal à 1 ou 2, en présence d'un catalyseur d'hydrogénation-déshydrogénation, à une température comprise entre 150 et 250°C, de préférence entre 175 et 220°C,

est caractérisé en ce que

l'opération d'ammonolyse est réalisée par mise en contact du ou des agents d'ammonolyse avec un mélange contenant le monoéther d'alkylèneglycol de formule II et le catalyseur d'hydrogénation-déshydrogénation, la quantité dudit catalyseur étant comprise entre 10 et 40% en poids par rapport au poids de monoéther d'alkylèneglycol, et en ce que l'on sépare la tris(éther-amine) formée.

La présence de catalyseur d'hydrogénation-déshydrogénation en quantité suffisante est fondamentale pour le déroulement de la réaction dans le sens de la formation de tris(éther-amines) tertiaires. Cette quantité de catalyseur est préférentiellement comprise entre 10 et 30% du poids de monoéther d'alkylèneglycol.

Bien que les catalyseurs classiques d'hydrogénation-déshydrogénation puissent être mis en oeuvre, ou préférera toutefois les catalyseurs au nickel, du type nickel Raney ou Harshaw.

Pour une bonne réalisation du procédé faisant l'objet de l'invention, on effectue l'opération d'ammonolyse de préférence en présence d'hydrogène, à une pression inférieure à 20 bars, et généralement à pression autogène, sous agitation violente jusqu'à disparition du monoéther d'alkylèneglycol; cette opération dure de 2 à 10 heures et généralement de 3 à 8 heures.

Parmi les monoéthers d'alkylèneglycol pouvant être mis en oeuvre, on peut citer:

— l'oxa-3 butanol-1
— le dioxa-3,6 heptanol-1
— le trioxa-3,6,9 décanol-1
— l'oxa-3 pentanol-1
— le dioxa-3,6 octanol-1
— le trioxa-3,6,9 undécanol-1
— l'oxa-3 hexanol-1
— le dioxa-3,6 nonanol-1
— le trioxa-3,6,9 dodécanol-1
— l'oxa-3 heptanol-1
— le dioxa-3,6 décanol-1
— le trioxa-3,6,9 tridécanol-1
— le phénoxy-5 oxa-3 pentanol-1
— le phénoxy-8 dioxa-3,6 octanol-1
— le cyclohexoxy-5 oxa-3 pentanol-1
— le cyclohexoxy-8 dioxa-3,6 octanol-1
— le nonylphénoxy-5 oxa-3 pentanol-1
— le nonylphénoxy-8 dioxa-3,6 octanol-1
— le dodécylphénoxy-5 oxa-3 pentanol-1
— le dodécylphénoxy-8 dioxa-3,6 octanol-1
— le dioxa-3,6 méthyl-4 heptanol-1
— le dioxa-3,6 diméthyl-2,4 heptanol-1

Parmi les ether-amines secondaires où primaires de formule (I') pouvant être mises en oeuvre, on peut citer:

Comme amines secondaires

— 1'aza-5 dioxa-2,8 nonane
— l'aza-8 tétraoxa-2,5,11,14 pentadécane
— l'aza-11 hexaoxa-2,5,8,14,17,20 uneicosane
— l'aza-6 dioxa-3,9 undécane

— l'aza-10 tétraoxa-4,7,13,16 nonadécane
— l'aza-9 tétraoxa-3,6,12,15 heptadécane
— l'aza-12 hexaoxa-3,6,9,15,18,21 tricosane
— l'aza-7 dioxa-4,10 tridécane
— l'aza-13 hexaoxa-4,7,10,16,19,22 pentacosane
— l'aza-8 dioxa-5,11 pentadécane
— l'aza-11 tétraoxa-5,8,14,17 uneicosane
— l'aza-14 hexaoxa-5,8,11,17,20,23 heptacosane
— l'aza-6 oxa-3 phenoxy-1 undécane
— l'aza-9 dioxa-3,6 phénoxy-1 heptadécane
— l'aza-6 oxa-3 cyclohexoxy-1 undécane
— l'aza-9 dioxa-3,6 cyclohexoxy-1 heptadécane
— l'aza-6 oxa-3 nonylphénoxy-1 undécane
— l'aza-9 dioxa-3,6 nonylphénoxy-1 heptadécane
— l'aza-6 oxa-3 dodécylphénoxy-1 undécane
— l'aza-9 dioxa-3,6 dodécylphénoxy heptadécane
— l'aza-8 tétraoxa-2,5,11,14 diméthyl-4,12 pentadécane
— l'aza-8 tétraoxa-2,5,11,14 tétraméthyl-4,6,10,12 pentadécane

Comme amines primaires
— l'oxa-3 butylamine
— l'oxa-3 pentylamine
— l'oxa-3 hexylamine
— l'oxa-3 heptylamine
— la dioxa-3,6 heptylamine
— la trioxa-3,6,9 undécylamine
— la dioxa-3,6 octylamine
— la trioxa-3,6,9 dodécylamine
— la dioxa-3,6 nonylamine
— la trioxa-3,6,9 tridécylamine
— la dioxa-3,6 décylamine
— la trioxa-3,6,9 tétradécylamine
— la phénoxy-5 oxa-3 pentylamine
— la phénoxy-8 dioxa-3,6 octylamine
— la cyclohexoxy-5 oxa-3 pentylamine
— la cyclohexoxy-8 dioxa-3,6 octylamine
— la nonylphénoxy-5 oxa-3 pentylamine
— la nonylphénoxy-8 dioxa-3,6 octylamine
— la dodécylphénoxy-5 oxa-3 pentylamine
— la dodécylphénoxy-8 dioxa-3,6 octylamine
— la dioxa-3,6 méthyl1-4 heptylamine
— la dioxa-3,6 diméthyl-2,4 heptylamine

Lorsque l'agent d'ammonolyse utilisé est l'ammoniac, celui-ci peut être mis en oeuvre selon des quantités qui ne sont pas aritiques; il y a toutefois intérêt à opérer en présence d'un large excès d'ammoniac par rapport à la quantité stoechiométriquement requise pour l'obtention d'éther-amines tertiaires, par exemple en présence d'au moins 2 fois cette quantité stoechiométrique et de préférence entre 2 et 5 fois cette quantité stoechiométrique.

La quantité d'hydrogène pouvant être mise en oeuvre n'est également pas critique; il y a intérêt à mettre en oeuvre de l à 50% en poids d'hydrogène par rapport au poids d'ammoniac et de préférence de 2 à 30% du poids d'ammoniac mis en oeuvre.

Selon une variante du procédé, on réalise l'opération d'ammonolyse sous courant d'ammoniac et d'hydrogène jusqu'à ce que 50 à 66% environ du monoéther d'alkylèneglycol soient transformés en amines, puis on laisse l'opération se terminer sous courant d'hydrogène seul.

Lorsque l'agent d'ammonolyse mis en oeuvre est l'éther-amine secondaire ou primaire correspondant à la tris(éther-amine) recherchée, il sera généralement favourable d'opérer avec un rapport molaire monoéther d'alkylèneglycol/éther-amine secondaire ou primaire d'au moins 1,3 fois le rapport stoechiométriquement requis pour l'obtention d'une tris(éther-amine); ce rapport pourra être généralement de 1,4 à 4 fois la stoechiométrie.

La quantité d'hydrogène pouvant alors être utilisée est de 1 à 10% du poids de monoéther d'alkylèneglycol mis en oeuvre, et de préférence de 1 à 5%.

L'eau de réaction formée en cours de réaction d'ammonolyse doit être éliminée du milieu réactionnel à l'aide d'un courant gazeux.

Lorsque de l'ammoniac et/ou de l'hydrogène sont mis en oeuvre, l'eau est entrainée par ce ou ces gaz; lorsqu'on opère sous pression autogène, une légère fuite de gaz permettra à la fois de régler la pression et d'éliminer l'eau formée; lorsqu'aucun gaz n'est mis en oeuvre, l'eau de réaction pourra être entrainée à l'azote par exemple.

L'opération d'ammonolyse est suivie d'une étape de séparation de la tris(éther-amine) formée· cette étape peut être réalisée par tout moyen connu, par exemple par distillation.

Le procédé faisant l'objet de l'invention est de préférence réalisé en discontinu; il peut toutefois

être réalisé en continu puisque les produits non complètement transformés en amines tertiaires correspondent aux éther-amines secondaires et/ou primaires pouvant être utilisées comme agent d'ammonolyse; en outre, le catalyseur conserve son activité et peut être recyclé.

Le procédé faisant l'objet de l'invention est tout particulièrement adapté à la préparation d'une manière sélective de tris (éther-amines) telles que:

— la tris(oxa-3 butyl)amine
— la tris(dioxa-3,6 heptyl)amine
— la tris(trioxa-3,6,9 décyl)amine
— la tris(oxa-3 pentyl)amine
— la tris(dioxa-3,6 octyl)amine
— la tris(trioxa-3,6,9 undécyl)amine
— la tris(oxa-3 hexyl)amine
— la tris(dioxa-3,6 nonyl)amine
— la tris(trioxa-3,6,9 dodécyl)amine
— la tris(oxa-3 heptyl)amine
— la tris(dioxa-3,6 décyl)amine
— la tris(trioxa-3,6,9 tridécyl)amine
— la tris(dioxa-3,6 méthyl-4 heptyl)amine
— la tris(dioxa-3,6 diméthyl-2,4 heptyl)amine
— la tris(phénoxy-5 oxa-3 pentyl)amine
— la tris(phénoxy-8 dioxa-3,6 octyl)amine
— la tris(cyclohexoxy-5 oxa-3 pentyl)amine
— la tris(cyclohexoxy-8 dioxa-3,6 octyl)amine
— la tris(nonylphénoxy-5 oxa-3 pentyl)amine
— la tris(nonylphénoxy-8 dioxa-3,6 octyl)amine
— la tris(dodécylphénoxy-5 oxa-3 pentyl)amine
— la tris(dodécylphénoxy-8 dioxa-3,6 octyl)amine

La présente invention a également pour objet les tris(polyoxaalcoyl)amines obtenues selon le procédé de l'invention.

Ces amines tertiaires peuvent être utilisées comme agents séquestrants pour solubiliser les sels métalliques organiques ou minéraux dans des solvants organiques dans lesquels ils ne sont pas solubles ou pour augmenter la solubilité des sels métalliques organiques ou minéraux dans les solvants organiques (demande de brevet français n° 79.05438 déposée le 2 Mars 1979 au nom de RHONE-POULENC INDUSTRIES). Ces amines tertiaires peuvent également être utilisées comme émulsifiants.

Les exemples suivants sont donnés à titre indicatif et ne peuvent être considérés comme une limite du domaine et de l'esprit de l'invention.

Example 1

Préparation de la tris(dioxa-3,6 octyl)amine de formule

$$N(CH_2—CH_2—O—CH_2—CH_2—O—C_2H_5)_3.$$

Dans un ballon tetracol, équipé d'une agitation, d'une arrivée d'ammoniac et d'hydrogène, d'une colonne et d'un condenseur pour recueillir l'eau entrainée par le courant gazeux, on charge:

— 195 g de Ni Raney déshydraté
et — 1400 g de dioxa-3,6 octanol(monoéther éthylique du diéthylèneglycol).

On porte la bouillie obtenue à 150°C, température à laquelle on envoie un courant gazeux composé de 51 g d'ammoniac et 2 g d'hydrogène à l'heure. On chauffe alors à 185°C, température que l'on maintient 3 heures. Environ 60% de l'éther d'alkylèneglycol ont été transformés en amines. On coupe ensuite l'arrivée d'ammoniac, tout en maintenant le courant d'hydrogène pendant 2 nouvelles heures à 185°C.

Après refroidissement on filtre le nickel Raney puis on distille le filtrat sous vide.

On obtient:

— 800 g de tris(dioxa-3,6 octyl)amine ayant un point d'ébullition de 195°C sous 0,5 mm de mercure,
— et 180 g de aza-9 tétraoxa-3,6,12,15 heptadécane de $Eb_{0,5} = 143°C$.

Le taux de transformation du dioxa-3,6 octanol est de 95%; les rendements sont de:

— 63% de tris(dioxa-3,6) octyl amine
— 14% en aza-9 tétraoxa-3,6-12,15 heptadécane
— le reste étant du diéthoxyéthane

L-opération d'ammonolyse décrite ci-dessus est réalisée en faisant varier les différentes quantités de Nickel Raney mises en oeuvre.

Les résultats obtenus après une même durée de réaction sont les suivants:

| | | | | | |
|---|---|---|---|---|---|
| % en poids de Ni par rapport a l'éther-alcool | 3 | 6 | 8,6 | 13,9 | 16 |
| taux de transformation de l'éther-alcool (%) | 98 | 94 | 96 | 95 | 96 |
| rendement en amine tertiaire (%) | 2 | 5 | 23 | 63 | 61 |
| rendement en amine secondaire (%) | 75 | 68 | 54 | 14 | 16 |

Ce tableau montre l'importance sur le déroulement de la réaction de la quantité de catalyseur mise en oeuvre.

### Exemple 2

Préparation de la tris(trioxa-3,6,9 undécyl)amine de formule

$$N(CH_2—CH_2—O—CH_2—CH_2—O—CH_2—CH_2—OC_2H_5)_3$$

On charge dans un ballon tétracol:

— 133 g d'aza-12 hexaoxa-3,6,9,15,18,21 tricosane de formule

$$NH(CH_2—CH_2—O—CH_2—CH_2—O—CH_2—CH_2—OC_2H_5)_2$$

— 20 g de nickel Raney

— et 180 g de trioxa-3,6,9 undécanol.

Le mélange est chauffé pendant 6 heures à 195°C sous un courant d'hydrogène de 2 g/h environ. Après filtration du nickel Raney, la tris(éther-amine) est distillée.

On obtient ainsi 156 g de tris(trioxa-3,6,9 undécyl)amine, soit un rendement de 66% par rapport à l'éther d'alkylèneglycol.

### Exemple 3

Préparation de la tris(dioxa-3,6 heptyl)amine de formule:

$$N(CH_2—CH_2—O—CH_2—CH_2O—CH_3)_3$$

L'opération décrite à l'exemple I est réalisée à partir de:

— 7500 g de nickel Raney

— 25000 g de dioxa-3,6 heptanol-1

— 1700 g/h d'ammoniac

— 50 g/h d'hydrogène

Le taux de transformation de l'éther-alcool est de 90%.

Les rendements sont de:

— 50% de tris(dioxa-3,6 heptyl)amine de $Eb_{0.5} = 160°C$

— 20% de aza-8 tétraoxa-2,5,11,14 pentadécane de $Eb_{0.5} = 125°C$

### Exemple 4

Préparation de la tris(dioxa-3,6 décyl)amine de formule:

$$N(CH_2—CH_2—O—CH_2—CH_2—O—C_4H_9)_3$$

L'opération décrite à l'exemple I est réalisée à partir de:

— 150 g de nickel Raney

— 558 g de dioxa-3,6 décanol-1

— 34 g/heure d'ammoniac

— 4 g/h d'hydrogène

Le taux de transformation de l'éther-alcool est de 90%.

Les rendements sont de:

— 50% de tris(dioxa-3,6 décyl)amine de $Eb_{0.5} = 240°C$

— 22% de aza-11 tétraoxa-5,8,14,17 heneicosane de Eb = 160°C sous 0,6 mm de Hg.

Exemple 5

Préparation de la tris(dioxa-3,6 octyl)amine

On charge dans l'appareillage décrit à l'exemple 1:

— 300 g de nickel Harshaw
— 1270 g de dioxa-3,6 octanol-1

On porte la bouillie à 150°C, on envoie un courant gazeux constitué de:

— 68 g/h d'ammoniac
— 3 g/h d'hydrogène

On chauffe à 185°C et maintient cette température pendant 6 h 30 mn; le courant d'ammoniac est maintenu pendant toute l'opération.

Après refroidissement, filtration et distillation, le taux de transformation de l'éther-alcool est de 96%.

Les rendements sont de:

— 60% en tris(dioxa-3,6 octyl)amine
— 15% en aza-9 tétraoxa-3,6,12,15 heptadécane

Exemple 6

On charge dans l'appareillage décrit à l'exemple 1

— 150 g de nickel Raney
— 800 g d'aza-9 tétraoxa-3,6,12,15 heptadécane
— 700 g de dioxa-3,6 octanol

On chauffe pendant 4 heures à 185°C sous un courant de 2 g/h d'hydrogène.

Après refroidissement, filtration et distillation, le taux de transformation de l'éther-alcool est de 72%. Le rendement en tris (dioxa-3,6 octyl)amine est de 86%.

Exemple 7

On réalise l'opération décrite à l'exemple 1 à partir de:

— 250 g de nickel Raney
— 1250 g de dioxa-3,6 octanol
— 51 g/h d'ammoniac
— 2 g/h d'hydrogène

Après refroidissement, on laisse décanter le nickel et on soutire 950 g de liquide constitué de 60,5% de tris(dioxa-3,6 heptyl)amine et de 14,5% d'amine secondaire.

On recharge 25 g de nickel Raney neuf sur le pied de nickel restant dans l'appareil et 1250 g de dioxa-3,6 octanol.

On répète l'opération d'ammonolyse dans les mêmes conditions.,

Après refroidissement et décantation, on soutire 1100 g de liquide contenant 60,1% de tris(dioxa-3,6 heptyl)amine et 14,7% d'amine secondaire.

On recommence cette opération 5 fois; au bout de la 5ème fois, le rendement en tris(dioxa-3,6 heptyl)amine est encore de 59,2%.

**Revendications**

1. Procédé de préparation de tris(éther-amines) de formule I

$$(I) \quad N \left[ A-O \left( B-O \right)_n R \right]_3$$

où R représente:

— un radical alkyle contenant de 1 à 24 atomes de carbone,
— un radical cyclohexyle
— un radical phényle
— un radical alkylphényle dont le groupe alkyle contient de 1 à 12 atomes de carbone

A et B sont semblables ou différents et représentent un groupement alcanediyle linéaire contenant 2 ou 3 atomes de carbone, lesdits atomes de carbone pouvant être substitués par un radical méthyle ou éthyle

n représente un nombre entier compris entre 0 et 4, par ammonolyse en phase liquide d'un monoéther d'alkylèneglycol de formule (II)

$$(II) \quad HO—A—O—(B—O)_n R$$

où R, A, B et n ont la définition donnée ci-dessus à l'aide d'au moins un agent d'ammonolyse choisi parmi l'ammoniac et les éther-amines de formule (I')

$$(I)' \quad {}_{3-p}(HN\!-\!)\left[A' - O -\!\!\left(B' - O\right)_{n'}\! R'\right]_p$$

où R', A', B' et n' sont respectivement identiques à R, A, B et n et p est égal à 1 ou 2, en présence d'un catalyseur d'hydrogénation-déshydrogénation, à une température comprise entre 150 et 250°C,

caractérisé en ce que

l'opération d'ammonolyse est réalisée par mise en contact du ou des agents d'ammonolyse avec un mélange contenant le monoéther d'alkylène-glycol de formule II et le catalyseur d'hydrogénation-déshydrogénation la quantité dudit catalyseur étant comprise entre 10 et 40% en poids par rapport au poids de monoéther d'alkylèneglycol, et en ce que l'on sépare la tris(éther-amine) formée.

2. Procédé selon la revendication 1 dans lequel n représente un nombre entier entre 0 et 3 et R contient 1 à 12 atomes de carbone lorsqu'il représente un radical alkyle.

3. Procédé selon la revendication 1 ou la revendication 2 dans lequel l'opération d'ammonolyse est réalisée à une température comprise entre 175 et 220°C.

4. Procédé selon l'une quelconque des revendications précédentes caractérisé en ce que l'opération d'ammonolyse est réalisée en présence de 10 à 35% en poids de catalyseur d'hydrogénation déshydrogénation par rapport au poids de monoéther d'alkylèneglycol.

5. Procédé selon l'une quelconque des revendications précédentes caractérisé en ce que le catalyseur d'hydrogénation-déshydrogénation mis en oeuvre est un catalyseur au nickel.

6. Procédé selon l'une quelconque des revendications précédentes caractérisé en ce que le monoéther d'alkylèneglycol de formule II est choisi parmi:

— l'oxa-3 butanol-1
— le dioxa-3,6 heptanol-1
— le trioxa-3,6,9 décanol-1
— l'oxa-3 pentanol-1
— le dioxa-3,6 octanol-1
— le trioxa-3,6,9 undécanol-1
— l'oxa-3 hexanol-1
— le dioxa-3,6 nonanol-1
— le trioxa-3,6,9 dodécanol-1
— l'oxa-3 heptanol-1
— le dioxa-3,6 décanol-1
— le trioxa-3,6,9 tridécanol-1
— le phénoxy-5 oxa-3 pentanol-1
— le phénoxy-8 dioxa-3,6 octanol-1
— le cyclohexoxy-5 oxa-3 pentanol-1
— le cyclohexoxy-8 dioxa-3,6 octanol-1
— le nonylphénoxy-5 oxa-3 pentanol-1
— le nonylphénoxy-8 dioxa-3,6 octanol-1
— le dodécylphénoxy-5 oxa-3 pentanol-1
— le dodécylphénoxy-8 dioxa-3,6 octanol-1
— le dioxa-3,6 méthyl-4 heptanol-1
— le dioxa-3,6 diméthyl-2,4 heptanol-1

7. Procédé selon l'une quelconque des revendications précédentes caractérisé en ce que l'opération d'ammonolyse est réalisée en présence d'hydrogène.

8. Procédé selon l'une quelconque des revendications précédentes caractérisé en ce que l'agent d'ammonolyse est de l'ammoniac et qu'il est mis en oeuvre selon des quantités correspondant à au moins deux fois la quantité stoechiométrique.

9. Procédé selon la revendication 8 caractérisé en ce que la quantité d'ammoniac mise en oeuvre est comprise entre 2 et 5 fois la quantité stoechiométrique.

10. Procédé selon la revendication 8 ou la revendication 9 caractérisé en ce que la réaction d'ammonolyse est réalisée en présence de 1 à 50% en poids d'hydrogène par rapport à l'ammoniac.

11. Procédé selon la revendication 10 caractérisé en ce que la quantité d'hydrogène est comprise entre 2 et 30% du poids d'ammoniac.

12. Procédé selon l'une quelconque des revendications 10 et 11 caractérisé en ce que l'opération d'ammonolyse est réalisée sous courant d'ammoniac et d'hydrogène jusqu'à ce que 50 à 60% du monoéther d'alkylèneglycol soient transformés puis se termine sous courant d'hydrogène.

13. Procédé selon l'une quelconque des revendications 1 à 7 caractérisé en ce que lorsque l'agent d'ammonolyse est une éther-amine primaire ou secondaire, la quantité d'éther-amine primaire ou secondaire à mettre en oeuvre est telle que le rapport monoéther d'alkylèneglycol/éther-amine primaire ou secondaire est égal à au moins 1,3 fois le rapport stoechiométrique.

14. Procédé selon la revendication 13, caractérisé en ce que le rapport monoéther d'alkylène-glycol/éther-amine primaire ou secondaire est compris entre 1,4 et 4 fois le rapport stoechiométrique.

15. Procédé selon la revendication 13 ou la revendication 14 caractérisé en ce que la réaction d'ammonolyse effectuée à l'aide d'éther-amine primaire ou secondaire est réalisée en présence de 1 à 10% en poids d'hydrogène par rapport au poids de monoéther d'alkylèneglycol.

16. Procédé selon la revendication 15 caractérisé en ce que la quantité d'hydrogène est comprise entre 1 et 5% du poids de monoéther d'alkylèneglycol.

17. Procédé selon l'une quelconque des revendications 1 à 7 et 13 à 16 caractérisé en ce que l'éther amine primaire est choisie parmi:

— l'oxa-3 butylamine
— l'oxa-3 pentylamine
— l'oxa-3 hexylamine
— l'oxa-3 heptylamine
— la dioxa-3,6 heptylamine
— la trioxa-3,6,9 undécylamine
— la dioxa-3,6 octylamine
— la trioxa-3,6,9 dodécylamine
— la dioxa-3,6 nonylamine
— la trioxa-3,6,9 tridécylamine
— la dioxa-3,6 décylamine
— la trioxa-3,6,9 tétradécylamine
— la phénoxy-5 oxa-3 pentylamine
— la phénoxy-8 dioxa-3,6 octylamine
— la cyclohexoxy-5 oxa-3 pentylamine
— la cyclohexoxy-8 dioxa-3,6 octylamine
— la nonylphénoxy-5 oxa-3 pentylamine
— la nonylphénoxy-8 dioxa-3,6 octylamine
— la dodécylphénoxy-5 oxa-3 pentylamine
— la dodécylphénoxy-8 dioxa-3,6 octylamine
— la dioxa-3,6 méthyl-4 heptylamine
— la dioxa-3,6 diméthyl-2,4 heptylamine

18. Procédé selon l'une quelconque des revendications 1 à 7 et 13 à 16 caractérisé en ce que l'éther-amine secondaire mis en oeuvre est choisie parmi:

— l'aza-5 dioxa-2,8 nonane
— l'aza-8 tétraoxa-2,5,11,14 pentadécane
— l'aza-11 hexaoxa-2,5,8,14,17,20 uneicosane
— l'aza-6 dioxa-3,9 undécane
— l'aza-10 tétraoxa-4,7,13,16 nonadécane
— l'aza-9 tétraoxa-3,6,12,15 heptadécane
— l'aza-12 hexaoxa-3,6,9,15,18,21 tricosane
— l'aza-7 dioxa-4,10 tridécane
— l'aza-13 hexaoxa-4,7,10,16,19,22 pentacosane
— l'aza-8 dioxa-5,11, pentadécane
— l'aza-11 tétraoxa-5,8,14,17 uneicosane
— l'aza-14 hexaoxa-5,8,11,17,20,23 heptacosane
— l'aza-6 oxa-3 phenoxy-1 undécane
— l'aza-9 dioxa-3,6 phenoxy-1 heptadécane
— l'aza-6 oxa-3 cyclohexoxy-1 undécane
— l'aza-9 dioxa-3,6 cyclohexoxy-1 heptadécane
— l'aza-6 oxa-3 nonylphénoxy-1 undécane
— l'aza-9 dioxa-3,6 nonylphénoxy-1 heptadécane
— l'aza-6 oxa-3 dodécylphénoxy-1 undécane
— l'aza-9 dioxa-3,6 dodécylphénoxy heptadécane
— l'aza-8 tétraoxa-2,5,11,14 diméthyl-4,12 pentadécane
— l'aza-8 tétraoxa-2,5,11,14 tétraméthyl-4,6,10,12 pentadécane

19. Tris(éther-amines) obtenues selon le procédé décrit dans l'une quelconque des revendications précédentes.

**Patentansprüche**

1. Verfahren zur Herstellung von Tris(ätheraminen) der allgemeinen Formel I

$$(I) \qquad N \left[ A - O \left( B - O \right)_n R \right]_3$$

in der R

— eine Alkylgruppe mit 1 bis 24 Kohlenstoffatomen,
— eine Cyclohexylgruppe,
— eine Phenylgruppe oder
— eine Alkylphenylgruppe mit 1 bis 12 Kohlenstoffatomen im Alkylteil darstellt,

A und B gleich oder verschieden sind und jeweils für eine lineare Alkandiylgruppe enthaltend 2 oder 3 Kohlenstoffatome steht, wobei diese Kohlenstoffatome durch eine Methylgruppe oder Äthylgruppe substituiert sein können,
n eine ganze Zahl von 0 bis 4 ist,
mittels Ammonolyse in flüssiger Phase eines Alkylenglykol-monoäthers der allgemeinen Formel II

$$(ii) \qquad HO{-}A{-}O{-}\left(B{-}O\right)_n R$$

in der R, A, B und n die oben angegebene Bedeutung haben, mittels mindestens einem Ammonolyse-Mittel ausgewählt unter Ammoniak und den Ätheraminen der allgemeinen Formel I',

$$(I)' \qquad {}_{3-p}(H)N \left[ A' - O \left( B' - O \right)_{n'} R' \right]_p$$

in der R', A', B' und n' jeweils identisch sind mit R, A, B und n und p die Zahl 1 oder 2 bedeutet, in Gegenwart eines Katalysators für die Hydrierung-Dehydrierung, bei einer Temperatur im Bereich von 150 bis 250°C dadurch gekennzeichnet, daß die Ammonolyse durchgeführt wird durch in-Berührungbringen des oder der Mittel für die Ammonolyse mit einem Gemisch, welches den Alkylenglykolmonoäther der allgemeinen Formel II und den Katalysator für die Hydrierung-Dehydrierung enthält, wobei die Menge des Katalysators 10 bis 40 Gew.-% ausmacht, bezogen auf das Gewicht des Alkylenglykol-monoäthers, und daß man das entstandene Tris(ätheramin) abtrennt.

2. Verfahren nach Anspruch 1, dadurch gekennzeichnet, daß n eine ganze Zahl von 0 bis 3 ist, und R 1 bis 12 Kohlenstoffatome enthält, wenn es für eine Alkylgruppe steht.

3. Verfahren nach Anspruch 1 oder 2, dadurch gekennzeichnet, daß die Ammonolyse bei einer Temperatur von 175 bis 220°C durchgeführt wird.

4. Verfahren nach irgendeinem der vorangegangenen Ansprüche, dadurch gekennzeichnet, daß die Ammonolyse in Gegenwart von 10 bis 35 Gew.-% Katalysator für die Hydrierung-Dehydrierung, bezogen auf das Gewicht des Alkylenglykol-monoäthers, durchgeführt wird.

5. Verfahren nach irgendeinem der vorgangegangenen Ansprüche, dadurch gekennzeichnet, daß der eingesetzte Katalysator für die Hydrierung-Dehydrierung ein Nickelkatalysator ist.

6. Verfahren nach irgendeinem der vorgangegangenen Ansprüche, dadurch gekennzeichnet, daß der Alkylenglykol-monoäther der allgemeinen Formel II ausgewählt wird unter:

— 3-Oxa-butanol-1
— 3,6-Dioxa-heptanol-1
— 3,6,9-Trioxa-decanol-1
— 3-Oxa-pentanol-1
— 3,6-Dioxa-octanol-1
— 3,6,9-Trioxa-undecanol-1
— 3-Oxa-hexanol-1
— 3,6-Dioxa-nonanol-1
— 3,6,9-Trioxa-dodecanol-1
— 3-Oxa-heptanol-1
— 3,6-Dioxa-decanol-1
— 3,6,9-Trioxa-tridecanol-1
— 5-Phenoxy-3oxa-pentanol-1
— 8-Phenoxy-3,6-dioxa-octanol-1
— 5-Cyclohexoxy-3oxa-pentanol-1
— 8-Cyclohexoxy-3,6-dioxa-octanol-1
— 5-Nonylphenoxy-3oxa-pentanol-1

— 8-Nonylphenoxy-3,6-dioxa-octanol-1
— 5-Dodecylphenoxy-3oxa-pentanol-1
— 8-Dodecylphenoxy-3,6-dioxa-octanol-1
— 3,6-Dioxa-4-methyl-heptanol-1
— Dioxa-2,4-dimethyl-heptanol-1

7. Verfahren nach irgendeinem der vorgangegangenen Ansprüche, dadurch gekennzeichnet, daß die Ammonolyse in Gegenwart von Wasserstoff durchgeführt wird.

8. Verfahren nach irgendeinem der vorgangegangenen Ansprüche, dadurch gekennzeichnet, daß das Mittel für die Ammonolyse Ammoniak ist und daß dieser in Mengen entsprechend mindestens der zweifachen stöchiometrischen Menge eingesetzt wird.

9. Verfahren nach Ansprüch 8, dadurch gekennzeichnet, daß die eingesetzte Menge Ammoniak zwei- bis fünffache stöchiometrische Menge ist.

10. Verfahren nach Anspruch 8 oder Anspruch 9, dadurch gekennzeichnet, daß die Ammonolysereaktion in Gegenwart von 1 bis 50 Gew.-% Wasserstoff, bezogen auf den Ammoniak, durchgeführt wird.

11. Verfahren nach Anspruch 10, dadurch gekennzeichnet, daß die Wasserstoffmenge 2 bis 30%, bezogen auf das Gewicht des Ammoniaks, ausmacht.

12. Verfahren nach einem der Ansprüche 10 und 11, dadurch gekennzeichnet, daß die Ammonolyse unter einem Ammoniak- und Wasserstoffstrom durchgeführt wird, bis 50 bis 60% des Alkylenglykol-monoäthers umgewandelt sind und dann unter einem Wasserstoffstrom zu Ende geführt wird.

13. Verfahren nach irgendeinem der Ansprüche 1 bis 7, dadurch gekennzeichnet, daß, wenn das Ammonolysemittel ein primäres oder sekundäres Ätheramin ist, die einzusetzende Menge an primärem oder sekundärem Ätheramin so beschaffen ist, daß das Verhältnis von Alkylenglykol-monoäther zu primärem oder sekundärem Ätheramin mindestens das 1,3-fache des stöchiometrischen Verhältnisses beträgt.

14. Verfahren nach Anspruch 13, dadurch gekennzeichnet, daß das Verhältnis von Alkylenglykol-monoäther zu primärem oder sekundärem Ätheramin das 1,4- bis 4-fache des stöchiometrischen Verhältnisses beträgt.

15. Verfahren nach Anspruch 13 oder Anspruch 14, dadurch gekennzeichnet, daß die mit Hilfe von primärem oder sekundärem Ätheramin durchgeführte Ammonolyse in Gegenwart von 1 bis 10 Gew.-% Wasserstoff, bezogen auf das Gewicht des Alkylenglykol-monoäthers ausgeführt wird.

16. Verfahren nach Anspruch 15, dadurch gekennzeichnet, daß die Wasserstoffmenge 1 bis 5 Gew.-%, bezogen auf den Alkylenglykol-monoäther, beträgt.

17. Verfahren nach irgendeinem der Ansprüche 1 bis 7 sowie 13 bis 16, dadurch gekennzeichnet, daß das primäre Ätheramin ausgewählt wird unter:

— 3-Oxa-gutylamin
— 3-Oxa-pentylamin
— 3-Oxa-hexylamin
— 3-Oxa-heptylamin
— 3,6-Dioxa-heptylamin
— 3,6,9-Trioxa-undecylamin
— 3,6-Dioxa-octylamin
— 3,6,9-Trioxa-dodecylamin
— 3,6-Dioxa-nonylamin
— 3,6,9-Trioxa-tridecylamin
— 3,6-Dioxa-decylamin
— 3,6,9-Trioxa-tetradecylamin
— 5-Phenoxy-3oxa-pentylamin
— 8-Phenoxy-3,6-dioxa-octylamin
— 5-Cyclohexoxy-3oxa-pentylamin
— 8-Cyclohexoxy-3,6-dioxa-octylamin
— 5-Nonylphenoxy-3oxa-pentylamin
— 8-Nonylphenoxy-3,6-dioxa-octylamin
— 5-Dodecylphenoxy-3oxa-pentylamin
— 8-Dodecylphenoxy-3,6-dioxa-octylamin
— 3,6-Dioxa-4-methyl-heptylamin
— 3,6-Dioxa-2,4-dimethyl-heptylamin

18. Verfahren nach irgendeinem der Ansprüche 1 bis 7 sowie 13 bis 16, dadurch gekennzeichnet, daß das eingesetzte sekundäre Ätheramin ausgewählt wird unter:

— 5-Aza-2,8-dioxa-nonan
— 8-Aza-2,5,11,14-tetraoxa-pentadecan
— 11-Aza-2,5,8,14,17,20-hexaoxa-uneicosan
— 6-Aza-3,9-dioxa-undecan
— 10-Aza-4,7,13,16-tetraoxa-nonadecan

— 9-Aza-3,6,12,15-tetraoxa-heptadecan
— 12-Aza-3,6,9,15,18,21-hexaoxa-tricosan
— 7-Aza-4,10-dioxa-tridecan
— 13-Aza-4,7,10,16,19,22-hexaoxa-pentacosan
— 8-Aza-5,11-dioxa-pentadecan
— 11-Aza-5,8,14,17-tetraoxa-uneicosan
— 14-Aza-5,8,11,17,20,23-hexaoxa-heptacosan
— 6-Aza-3oxa-1-phenoxy-undecan
— 9-Aza-3,6-dioxa-1-phenoxy-heptadecan
— 6-Aza-3oxa-1-cyclohexocy-undecan
— 9-Aza-3,6-dioxa-1-cyclohexoxy-heptadecan
— 6-Aza-3oxa-1-nonylphenoxy-undecan
— 9-Aza-3,6-dioxa-1-nonylphenoxy-heptadecan
— 6-Aza-3oxa-1-dodecylphenoxy-undecan
— 9-Aza-3,6-dioxa-dodecylphenoxy-heptadecan
— 8-Aza-2,5,11,14-tetraoxa-4,12-dimethyl-pentadecan
— 8-Aza-2,5,11,14-tetraoxa-4,6,10,12-tetramethyl-pentadecan

19. Tris(ätheramine) erhalten nach dem in irgendeinem der vorangegangenen Ansprüche beschriebenen Verfahren.

## Claims

1. Process for the preparation of tris-(ether-amines) of the formula I

$$(I) \quad N \left[ A-O \left( B-O \right)_n R \right]_3$$

in which R represents: an alkyl radical containing from 1 to 24 carbon atoms, a cyclohexyl radical, a phenyl radical or an alkylphenyl radical in which the alkyl group contains from 1 to 12 carbon atoms, A and B are similar or different and represent a linear alkanediyl group containing 2 or 3 carbon atoms, it being possible for these carbon atoms to be substituted by a methyl or ethyl radical, and n represents an integer between 0 and 4, by ammonolysis, in the liquid phase, of an alkylene glycol monoether of the formula (II)

$$(II) \quad HO-A-O-(B-O)_n R$$

in which R, A, B and n have the definition given above, with the aid of at least one ammonolysis agent chosen from amongst ammonia and the ether-amines of the formula (I')

$$(I)' \quad {}_{3-p}H-N \left[ A'-O \left( B'-O \right)_{n'} R' \right]_p$$

in which R', A', B' and n' are respectively identical to R, A, B and n and p is equal to 1 or 2, in the presence of a hydrogenation/dehydrogenation catalyst, at a temperature of between 150 and 250°C, characterised in that the ammonolysis operation is carried out by bringing the ammonolysis agent or agents into contact with a mixture containing the alkylene glycol monoether of the formula II and the hydrogenation/dehydrogenation catalyst, the amount of the said catalyst being between 10 and 40% by weight, relative to the weight of alkylene glycol monoether, and in that the tris-(ether-amine) formed is separated off.

2. Process according to Claim 1, in which n represents an integer between 0 and 3 and R contains 1 to 12 carbon atoms if it represents an alkyl radical.

3. Process according to Claim 1 or Claim 2, in which the ammonolysis operation is carried out at a temperature between 175 and 220°C.

4. Process according to any one of the preceding claims, characterised in that the ammonolysis operation is carried out in the presence of 10 to 35% by weight of hydrogenation/dehydrogenation catalyst, relative to the weight of alkylene glycol monoether.

5. Process according to any one of the preceding claims, characterised in that the hydrogenation/dehydrogenation catalyst used is a nickel catalyst.

6. Process according to any one of the preceding claims, characterised in that the alkylene glycol monoether of the formula II is chosen from amongst: 3-oxa-butan-1-ol, 3,6-dioxaheptan-1-ol, 3,6,9-trioxadecan-1-ol, 3-oxapentan-1-ol, 3,6-dioxaoctan-1-ol, 3,6,9-trioxaundecan-1-ol, 3-oxahexan-1-ol 3,6-dioxanonan-1-ol, 3,6,9-trioxadodecan-1-ol, 3-oxaheptan-1-ol, 3,6-dioxadecan-1-ol, 3,6,9-trioxa-tridecan-1-ol, 5-phenoxy-3-oxapentan-1-ol, 8-phenoxy-3,6-dioxaoctan-1-ol, 5-cyclohexyloxy-3-oxa-

pentan-1-ol, 8-cyclohexyloxy-3,6-dioxaoctan-1-ol, 5-nonylphenoxy-3-oxapentan-1-ol, 8-nonylphenoxy-3,6-dioxaoctan-1-ol, 5-dodecylphenoxy-3-oxapentan-1-ol, 8-dodecylphenoxy-3,6-dioxaoctan-1-ol, 3,6-dioxa-1,4-methylheptan-1-ol and 3,6-dioxa-1,2,4-dimethylheptan-1-ol.

7. Process according to any one of the preceding claims, characterised in that the ammonolysis operation is carried out in the presence of hydrogen.

8. Process according to any one of the preceding claims, characterised in that ammonolysis agent is ammonia and in that it is used in amounts corresponding to twice the stoichiometric amount.

9. Process according to Claim 8, characterised in that the amount of ammonia used is between 2 and 5 times the stoichiometric amount.

10. Process according to Claim 8 or Claim 9, characterised in that the ammonolysis reaction is carried out in the presence of 1 to 50% by weight of hydrogen, relative to the ammonia.

11. Process according to Claim 10, characterised in that the amount of hydrogen is between 2 and 30% of the weight of ammonia.

12. Process according to either one of Claims 10 and 11, characterised in that the ammonolysis operation is carried out under a stream of ammonia and hydrogen until 50 to 60% of the alkylene glycol monoether has been converted, and is then completed under a stream of hydrogen.

13. Process according to any one of Claims 1 to 7, characterised in that, if the ammonolysis agent is a primary or secondary ether-amine, the amount of primary or secondary either-amine to be used is such that the ratio alkylene glycol monoether/primary or secondary ether-amine is equal to at least 1.3 times the stoichiometric ratio.

14. Process according to Claim 13, characterised in that the ratio alkylene glycol monoether/primary or secondary either-amine is between 1.4 and 4 times the stoichiometric ratio.

15. Process according to Claim 13 or Claim 14, characterised in that the ammonolysis reaction effected with the aid of primary or secondary ether-amine is carried out in the presence of 1 to 10% by weight of hydrogen, relative to the weight of alkylene glycol monoether.

16. Process according to Claim 15, characterised in that the amount of hydrogen is between 1 and 5% of the weight of alkylene glycol monoether.

17. Process according to any one of Claims 1 to 7 and 13 to 16, characterised in that the primary ether-amine is chosen from amongst: 3-oxabutylamine, 3-oxapentylamine, 3-oxahexylamine, 3-oxaheptylamine, 3,6-dioxaheptylamine, 3,6,9-trioxaundecylamine, 3,6-dioxaoctylamine, 3,6,9-trioxadodecylamine, 3,6-dioxanonylamine, 3,6,9-trioxatridecylamine, 3,6-dioxadecylamine, 3,6,9-trioxatetradecylamine, 5-phenoxy-3-oxapentylamine, 8-phenoxy-3,6-dioxaoctylamine, 5-cyclohexyloxy-3-oxapentylamine, 8-cyclohexyloxy-3,6-dioxaoxtylamine, 5-nonylphenoxy-3-oxapentylamine, 8-nonylphenoxy-3,6-dioxaoctylamine, 5-dodecylphenoxy-3-oxapentylamine, 8-dodecylphenoxy-3,6-dioxaoctylamine, 3,6-dioxa-1-4-methylheptylamine and 3,6-dioxa-2,4-dimethylheptylamine.

18. Process according to any one of Claims 1 to 7 and 13 to 16, characterised in that the secondary ether-amine used is chosen from amongst: 5-aza-2,8-dioxanonane, 8-aza-2,5,11,14-tetraoxapentadecane, 11-aza-2,5,8,17,20-hexaoxauneicosane, 6-aza-3,9-dioxaundecane, 10-aza-4,7,13,16-tetraoxanonadecane, 9-aza-3,6,12,15-tetraoxaheptadecane, 12-aza-3,6,9,15,18,21-hexaoxatricosane, 7-aza-4,10-dioxatridecane, 13-aza-4,7,10,16,19,22-hexaoxapentacosane, 8-aza-5,11-dioxapentadecane, 11-aza-5,8,14,17-tetraoxauneicosane, 14-aza-5,8,11,17,20,23-hexaoxaheptacosane, 6-aza-3-oxa-1-phenoxyundecane, 9-aza-3,6-dioxa-1-phenoxy-heptadecane, 6-aza-3-oxa-1-cyclohexyloxyundecane, 9-aza-3,6-dioxa-1-cyclohexyloxyheptadecane, 6-aza-3-oxa-1-nonylphenoxyundecane, 9-aza-3,6-dioxa-1-nonylphenoxyheptadecane, 6-aza-3-oxa-1-dodecylphenoxyundecane, 9-aza-3,6-dioxa-1-dodecylphenoxyheptadecane, 8-aza-2,5,11,14-tetraoxa-4,12-dimethylpentadecane and 8-aza-2,5,11,14-tetraoxa-4,6,10,12-tetramethylpentadecane.

19. Tris-(ether-amines) obtained in accordance with the process described in any one of the preceding claims.